(19) Europäisches Patentamt / European Patent Office / Office européen des brevets

(11) EP 1 674 134 B1

(12) EUROPÄISCHE PATENTSCHRIFT

(45) Veröffentlichungstag und Bekanntmachung des Hinweises auf die Patenterteilung:
**21.01.2009 Patentblatt 2009/04**

(21) Anmeldenummer: **05024374.0**

(22) Anmeldetag: **09.11.2005**

(51) Int Cl.:
*A61Q 19/10* (2006.01) *A61K 8/06* (2006.01)
*A61K 8/19* (2006.01) *A61K 8/46* (2006.01)
*A61K 8/81* (2006.01) *A61K 8/89* (2006.01)
*A61K 8/92* (2006.01)

(54) **Tensidhaltige Reinigungsemulsion mit festen Partikeln**

Surfactant-containing cleaning emulsion comprising solid particles

Emulsion de nettoyage à base de tensioactifs comprenant des particules solides

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.12.2004 DE 102004062771**

(43) Veröffentlichungstag der Anmeldung:
**28.06.2006 Patentblatt 2006/26**

(73) Patentinhaber: **Beiersdorf AG**
**20253 Hamburg (DE)**

(72) Erfinder:
- **Küther, Jörg**
**25469 Halstenbek (DE)**
- **Bluschke, Torsten**
**21717 Fredenbeck-Schwinge (DE)**

(56) Entgegenhaltungen:
**EP-A- 0 336 900** **WO-A-03/043599**
**WO-A-03/049715** **WO-A-03/095600**
**FR-A- 2 276 030** **FR-A- 2 731 616**
**US-A- 4 155 870** **US-A- 4 284 533**
**US-A- 4 508 634** **US-B1- 6 489 275**

Bemerkungen:
Die Akte enthält technische Angaben, die nach dem Eingang der Anmeldung eingereicht wurden und die nicht in dieser Patentschrift enthalten sind.

EP 1 674 134 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft tensidhaltige Reinigungsemulsion mit festen Partikeln, insbesondere Duschprodukte.

Menschliche Haut unterliegt einem kontinuierlichem natürlichen Erneuerungsprozess bei dem fortlaufend abgestorbene Hautzellen von der Hautoberfläche abgeschilfert werden. Verbleiben abgestorbene Hautzellen auf der Oberfläche kann dies über ein unebenes Hautbild hinaus, zu Schuppenbildung bis hin zu krankhaften Veränderungen des Hautbildes führen.

Zur Unterstützung des natürlichen Abschilferungsprozesses sind kosmetische oder medizinische Zubereitungen in Form von Gelen, Pasten, Creme oder Salben bekannt, die Schleifpartikel enthalten. Besonders beliebt sind solche Formulierungen in Form von Duschgelen.

Wesentlicher Nachteil aller bisher bekannter Duschformulierungen ist ein Mangel an Hautpflege. Durch den mechanischen Stress der Schleifpartikel und des Reinigungsprozesses wird die Haut trocken und verliert Lipide. Dies kann insbesondere bei häufiger Anwendung zu ernsthaften Störungen des physiologischen Hautgleichgewichtes führen.

Bisher sind keine Duschprodukte in Form von tensidhaltigen Emulsionen mit hohen Lipidanteilen und festen Schleifpartikeln bekannt. Eine besondere Herausforderung besteht zudem darin, diese Partikel langfristig stabil in Schwebe zu halten.

Schleifpartikel im Sinne der vorliegenden Schrift sind feste, abrasive Partikel aus allen organischen und anorganischen Feststoffen auf natürlicher und synthetischer Basis. Verwendet werden zum Beispiel Kunstoffpartikel aus beispielhaft Viskose, Zellulose, Polypropylen, Polyester, Polyethylenterephthalat (PET), Polytetrafluorethylen (PTFE), Aramid, Nylon, Kevlar, Polyvinylderivate, Polyurethane, Polylactid, Polyhydroxyalkanoat, Polycarbonat, Polystyrol, Celluloseester und/oder Polyethylen, sowie schwer- bzw. unlösliche Kristalle wie z.B. Kalziumsulfat, Kalziumcarbonat, verkapselte oder beschichtete Kristalle wie z.B. Natriumchlorid, Kalziumchlorid, Kaliumchlorid, Magnesiumchlorid, Zukker, Silikate wie z.B. Seesand, Tonerde, Schlämme, geschrotete oder gemahlene Naturprodukte wie z.B. Weizen, Leinsamen, Reis, Mais, Mandeln, Nüsse, Nussschalen, Kürbiskerne, Kümmel, geschrotete oder gemahlene natürliche Schwämme wie z.B. Lufagurke, natürliche und syntetische Wachse wie z.B. Reiskleiewachs, Caranaubawachs, Jojobawachs, Bienenwachs.

Filmbildner im Sinne der vorliegenden Schrift sind: polymere quaternäre Ammoniumverbindungen z.B. Polyquaternium-2, Polyquaternium-5, Polyquaternium-6, Polyquaternium-7, Polyquaternium-10, Polyquaternium-11, Polyquaternium-16, Polyquaternium-17 sowie Cellulosederivate und quaternisierte Guar Gum Derivate z.B. Jaguar Excel®, Jaguar C 162® der Firma Rhodia.

Als Fließgrenze im Sinne der vorliegenden Schrift wird die kritische Schubspannung der Fließkurve angesehen. Gemessen wird die Fließkurve auf einem schubspannungsgesteuerten Rheometer Typ SR-2000 der Firma Rheometric Scientific bei 25°C $\pm$ 1°C mit 25 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Das Produkt wird vorsichtig mit einem Löffelspatel entnommen und in das Messgerät eingebracht. Dabei sollte das Produkt nicht geschert werden, um eine Zerstörung oder Beeinflussung der Strukturen zu vermeiden.

Unter einem tan delta im Sinne der vorliegenden Schrift wird der Quotient aus dem Verlustmodul und dem Speichermodul verstanden.

Gemessen werden Verlust- und Speichermodul durch einen dynamischen Frequenztest auf einem schubspannungsgesteuerten Rheometer bei 40°C $\pm$ 1°C mit 25 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan delta im Frequenzbereich zwischen 0,05 rad/s und 3,0 rad/s angegeben, bevorzugt zwischen 0,08 rad/s und 1,0 rad/s.

Unter dem Partikel-Sedimentationskoeffizient im Sinne der vorliegenden Schrift wird das Verhältnis zwischen homogen verteilten und sedimentierten Partikeln verstanden.

Gemessen wird der Anteil an Partikeln die nach einer 4 monatigen Lagerzeit bei 50°C zusätzlich sedimentiert sind. Dazu wird zu Beginn (A) und am Ende (E) der Lagerzeit eine Probe aus dem unteren Viertel der zu lagernden Masse genommen, in der jeweils der Anteil an festen Partikeln mit gravimetrischen Methoden bestimmt wird.

Zur Berechnung des Partikel-Sedimentationskoeffizient dient folgende Formel:

$$K_{sed} = \frac{C_{(E)}}{C_{(A)}}$$

Ungesättigte Lipide im Sinne der vorliegenden Schrift sind Beispielsweise Olivenöl, Sonnenblumenöl, Sojaöl, Erdnussöl, Reisöl, Rapsöl, Mandelöl, Palmöl, Ricinusöl, Kokosöl, Palmkernöl.

Keratinische Strukturen im Sinne der vorliegenden Schrift sind Haut, Haar, Nägel, Hufe von Menschen oder Säugetieren.

Stand der Technik:

DE 10318526 offenbart eine Reinigungsemulsion enthaltend Natriumlaurethsulfat und/oder Natriummyrethsulfat in einer Gesamtkonzentration von 2 bis 17 Gewichts-%, ein oder mehrere Polyacrylate, welche gewählt werden aus der Gruppe, die gebildet wird aus anionischen Homo- und/oder Copolymeren der Acrylsäure und/oder alkylierten Acrylsäurederivaten sowie deren Estern, in einer Gesamtkonzentration von 0,20 bis 0,74 Gewichts-% eine Ölphase in einer Gesamtkonzentration von 43-51 Gewichts-%, enthaltend Paraffinöl in einer Konzentration von 25 bis 50 Gewichts-% und ein oder mehrere Öle mit einer Polarität von 5 bis 50 mN/m in einer Gesamtmenge von 1 bis 25 Gewichts-%, jeweils bezogen auf das Gesamtgewicht der Emulsion, neben gegebenenfalls weiteren kosmetischen und/oder dermatologischen Wirk-, Hilfs- und Zusatzstoffen, die eine Viskosität von 500 bis 3500 mPa s bei 100 1/s aufweist.

EP 1166772 offenbart kosmetische oder dermatologische Reinigungsemulsionen, die bezogen auf das Gesamtgewicht der Zubereitungen 1 bis 30 Gew.-% eines oder mehrerer waschaktiver Tenside, gewählt aus der Gruppe der Tenside, welchen einen HLB-Wert von mehr als 15 haben, 35 bis 50 Gew.-% einer oder mehrerer Ölkomponenten, 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und 5 bis 60 Gew.-% Wasser enthalten.

DE 10153023 offenbart Reinigungsemulsionen, die bezogen auf das Gesamtgewicht der Zubereitungen 1 bis 30 Gew.-% eines oder mehrerer waschaktiver Tenside, gewählt aus der Gruppe der Tenside, welchen einen HLB-Wert von mehr als 15 haben, 35 bis 50 Gew.-% einer oder mehrerer Ölkomponenten, 0,001 bis 30 Gew.-% eines oder mehrerer Wirkstoffe, 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und 5 bis 60 Gew.-% Wasser enthalten.

DE 10161171 offenbart kosmetische oder dermatologische Reinigungsemulsionen, die bezogen auf das Gesamtgewicht der Zubereitungen 1 bis 30 Gew.-% eines oder mehrerer waschaktiver Tenside, gewählt aus der Gruppe der Tenside, welchen einen HLB-Wert von mehr als 15 haben, 35 bis 50 Gew.-% einer oder mehrerer Ölkomponenten, 0,1 bis 10 Gew.-% Talkum, 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und 5 bis 60 Gew.-% Wasser enthalten.

WO 9632092, offenbart Zusammensetzungen in Form von O/W-Emulsionen, die frei von bestimmten 1-Alkenen sind und aus 0,01 bis 5 Gew.% bestimmter Copolymere von beispielsweise Acrylsäure, 0,05 bis 20 Gew.% eines nichtemulgiernden Waschtensides mit einem HLB-Wert grösser 11, 0,05 bis 40 Gew.% bestimmter Öle und 20 bis 99,44 Gew.% Wasser.

FR 2731616 offenbart eine Creme in Form einer o/w- Emulsion enthaltend Vaselinöl, wasser, ein anionisches Tensid und ein nichtion isches Tensid, ein Polyethy Penpulver als Schleif mittel, und 0,8 Gew.% eines Acrylat / $C_{10-30}$-Alhklacrylat Crosspolymers als verdicher.

Ausgehend von all dem fehlt es dem Stand der Technik an einem Produkt, das gute Pflegeeigenschaften und sehr gute Rückfettungseigenschaften bei gleichzeitig (guter Reinigungsleistung) effektiver Schleifleistung aufweist.

Bisher sind keine tensidhaltigen Emulsionen mit hohen Lipidanteilen und festen Peelingpartikeln bekannt, die sich als Duschprodukt eignen. Eine besondere Herausforderung besteht darin, die Partikel langfristig stabil in Schwebe zu halten.

Über solche stabilen, höherviskosen Emulsionen mit einem Gehalt an Schleifkörpem wird in den angegebenen Schriften nichts offenbart.

Es wurde nun gefunden, dass eine kosmetische Reinigungsemulsion enthaltend Öl, Wasser, Tenside, Schleifkörper und Verdicker wobei als Verdicker 0,3 bis 1,0 Gew.%; besonders bevorzugt 0,7 bis 1 Gew.% von Acryl- und/oder Methacrylsäure und/oder deren Derivaten abgeleitetes Polymer und als Schleifkörper solche mit mit einer durchschnittlichen Partikelgröße von 100μm - 1500μm bevorzugt 200μm - 1000μm besonders bevorzugt 300μm - 500μm verwendet werden, dadurch gekennzeichnet, dass sie mehr als 10 Gew.% Paraffinum Liquidum enthält, den Nachteilen des Standes der Technik abhilft. Dadurch werden insbesondere hohe Langzeitstabilität, gute Reinigungsleistung und gutes Hautgefühl erreicht.

Die Herstellung einer langzeitstabilen tensidhaltige Emulsion mit hohem Lipidanteil und festen Peelingpartikeln war überraschend, weil Prognosen über die Langzeitstabilität solcher Systeme schwer zu geben sind und insofern die Eigenschaften dieser Formulierung erstaunlich sind. Die erfindungsgemäße Zubereitung weist vielfache Vorteile auf:

- Neben den Pflege- und/ oder Reinigungseigenschaften stellt die Handhabung eines kosmetischen Produktes einen für den Verbraucher sehr wichtigen Punkt dar. Die hier beschriebene Formulierung verfügt über sehr gute Anwendungseigenschaften. Sie lässt sich leicht entnehmen und dosieren. Zudem ist sie auf der Haut gut verteilbar und lässt sich nach der Anwendung einfach abspülen.

- Das Produkt weist eine sehr gute Langzeitstabilität auf. Diese garantiert über den gesamten Verwendungszeitraum eine gleich bleibende Produktqualität und Produktleistung.

- Durch gute Schaum- und sehr gute Reinigungsleistung des Produktes ist die Anwendung eines zusätzlichen Körperreinigungsproduktes nicht mehr nötig.

- Sowohl objektiv nachvollziehbare als auch subjektiv spürbare Rückfettung generiert ein gepflegtes Hautgefühl. Dies ermöglicht Menschen mit normaler Haut auf zusätzliche rückfettende Körperpflegemittel, deren Einsatz nach Dusche und Bad sehr verbreitet ist, zu verzichten.

- In dieser Formulierung gelingt es eine Kombination von milden Reinigungssubstanzen, Rückfettung und abrasiven Partikeln zu erstellen, die sich durch einen hohen Grad an Milde auszeichnet.

- Der hier gewählte Formulierungstyp einer Emulsion weist eine sehr attraktive optische Anmutung für den Verbraucher auf.

Weiterhin ist es bevorzugt, wenn der Partikel-Sedimentationskoeffizient nach einer viermonatigen Lagerung bei 50°C weniger als 1,2 bevorzugt weniger als 1,1 beträgt. Dadurch wird die Langzeitstabilität des Produktes besonders positiv beeinflusst.
Weiterhin ist es bevorzugt, wenn Schleifkörper mit einem Schmelzpunkt unterhalb 700 °C verwendet werden, besonders bevorzugt mit einem Schmelzpunkt von 60 °C bis 200 °C. Dadurch wird die Zubereitung bei der Anwendung besonders hautfreundlich.
Weiterhin ist es bevorzugt, wenn die Schleifkörper mit einer Dichte größer 0,9 g $cm^{-3}$ , bevorzugt größer 0,9 kleiner 3, besonders bevorzugt größer 0,9 kleiner 1 verwendet werden, wodurch wieder insbesondere die Langzeitstabilität positiv beeinflusst wird.
Weiterhin ist es bevorzugt, wenn mindestens 0,5 Gew.%, bevorzugt mehr als 1,0 Gew.%, besonders bevorzugt mehr als 1,5 Gew.%, ganz besonders bevorzugt mehr als 3 Gew.% und höchstens 15 Gew.% , besonders bevorzugt höchstens 10 Gew.%, ganz besonders bevorzugt höchstens 5 Gew.% Schleifkörper verwendet werden. Dadurch werden insbesondere eine gute Reinigungsleistung und angenehmes Hautgefühl erreicht.
Weiterhin ist es bevorzugt, wenn als Verdicker ein Alkylacrylat-Polymer verwendet wird, besonders bevorzugt C10-C30 Alkylacrylatcrosspolymer. Dadurch wird insbesondere die Langzeitstabilität positiv beeinflusst.
Weiterhin ist es bevorzugt, wenn die Viskosität von 3600 mPas bis 15000 mPas, bevorzugt 4000 mPas bis 10000 mPas, besonders bevorzugt 4500 bis 7000 mPas beträgt, ermittelt mit Rotationsviskosimeter VIskotester VT02 der Firma Haake mit jeweils Messbereich adäquater Spindel. Bei diesem Gerät gibt es drei Messbereiche. Je nachdem wie hoch die Viskosität tatsächlich ist kann man dann nur mit der für den Messbereich vorgeschriebenen Spindel messen. Die Wahl der Spindel wird folglich durch die Viskosität des Produktes vorgegeben. Dadurch werden insbesondere die Anwendungseigenschaften positiv beeinflusst.
Weiterhin ist es bevorzugt, wenn die Fließgrenze 0,5 Pa bis 300 Pa, bevorzugt 0,5 bis 100 Pa und besonders bevorzugt 1 bis 60 Pa beträgt. Dadurch werden insbesondere die Anwendungseigenschaften positiv beeinflusst.
Weiterhin ist es bevorzugt, wenn die Zubereitung ein tan delta zwischen 0,01 und 0,8, bevorzugt zwischen 0,01 und 0,6 und besonders bevorzugt zwischen 0,1 bis 0,5 aufweist. Dadurch wird insbesondere die Langzeitstabilität positiv beeinflusst.
Weiterhin ist es bevorzugt, wenn die Zubereitung mindestens 10 Gew.%, bevorzugt mindestens 25 Gew.% , besonders bevorzugt mindestens 41 Gew.% und höchstens 50 Gew.%, besonders bevorzugt höchstens 47 Gew.% Öl enthält. Dadurch wird insbesondere die Rückfettung der Haut positiv beeinflusst.
Weiterhin ist es bevorzugt, wenn die Zubereitung mindestens 5 Gew.%, besonders bevorzugt mehr als 10 Gew.% und höchstens 15 Gew.% Tenside enthält. Dadurch werden insbesondere gute Schaumeigenschaften und eine sehr gute Reinigungsleistung erreicht. Weiterhin ist es bevorzugt, wenn die Emulsion mindestens ein anionisches Tensid enthält, besonders bevorzugt 5 bis 12 Gew.% enthält. Dadurch werden insbesondere gute Schaumeigenschaften und eine sehr gute Reinigungsleistung sowie ein hoher Mildegrad

erreicht. Die Emulsion enthätt mehr als 10 Gew.% Paraffinum Liquidum, bevorzugt enthätt sie 21-49 Gew.%. Dadurch wird insbesondere die Rückfettung, positiv beeinflusst und ein gutes Hautgefühl bewirkt.

Weiterhin ist es bevorzugt, wenn die Emulsion mehr als 5 Gew.% ungesättigter Lipide enthält. Dadurch wird

insbesondere die Rückfettung positiv beeinflusst.
Die Erfindung umfasst auch die Verwendung von solchen Emulsionen zur Reinigung und Pflege von keratinischen Strukturen.

Bevorzugt ist es wenn als Polyacrylat ein oder mehrere Polyacrylate, welche gewählt werden aus der Gruppe, die gebildet wird aus anionischen Homo- und/oder Copolymeren der Acrylsäure und/oder alkylierten Acrylsäurederivaten sowie deren Estern, verwendet wird.
Ebenso bevorzugt ist es, als Polyacrylat ein oder mehrere Polyacrylate, welche gewählt werden aus der Gruppe, die gebildet wird aus anionischen Copolymeren der Acrylsäure und/oder alkylierten Acrylsäurederivaten sowie deren Estern, verwendet wird.
Erfindungsgemäß bevorzugt sind Acrylat-Copolymere und/oder Acrylat-Alkylacrylat-Copolymere, welche unter den Handelbezeichnungen Carbopol. 1382, Carbopol. 981 und Carbopol. 5984, Aqua SF-1 von der NOVEON Inc. bzw. als Aculyn. 33 von International Specialty Products Corp. erhältlich sind. Erfindungsgemäß besonders bevorzugt sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit. Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Carbopol ETD 2020, Carbopol 3128, Pemulen TR1 und Pemulen TR2 bei der NOVEON Inc. erhältlichen.

Besonders bevorzugt ist es, wenn das Polyacrylat quervernetzt ist und ganz besonders bevorzugt mit einem C10-C30-Alkylrest

Es ist erfindungsgemäß vorteilhaft, den erfindungsgemäßen Reinigungszubereitungen Filmbildner (Konditionierer) zuzusetzen.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF).

Vorteilhafte fettlösliche Filmbildner sind z. B., die Filmbildner aus der Gruppe der Polymere auf Basis von Polyvinylpyrrolidon (PVP)

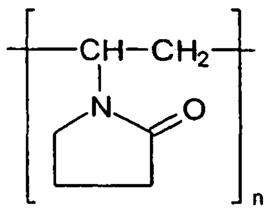

Vorteilhaft sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Als Filmbildner mit wenigstens einer teilweise quaternisierten Stickstoffgruppen eigenen sich bevorzugt solche, welche gewählt werden aus der Gruppe der Substanzen, welche nach der INCI-Nomenklatur (International Nomenclature Cosmetic Ingredient) den Namen "Polyquaternium" tragen, beispielsweise:

| | |
|---|---|
| Polyquaternium-2 | (Chemical Abstracts-Nr. 63451-27-4, z.B. Mirapol® A-15) |
| Polyquaternium-5 | (Copolymeres aus dem Acrylamid und dem β-Methacryloxyethyltri-methylammoniummethosulfat, CAS-Nr. 26006-22-4) |
| Polyquaternium-6 | (Homopolymer des N,N-Dimethyl-N-2-propenyl-2-propen-1-aminiumchlorids, CAS-Nr. 26062-79-3, z.B. Merquat® 100 |
| Polyquaternium-7 | N,N-Dimethyl-N-2-propenyl-2-propen-1-aminiumchlorid, Polymeres mit 2-Propenamid, CAS-Nr. 26590-05-6, z.B. Merquat® S |
| Polyquaternium-10 | Quaternäres Ammoniumsalz der Hydroxyethylcellulose, CAS-Nr. 53568-66-4, 55353-19-0, 54351-50-7, 68610-92-4, 81859-24-7, z.B. Celquat® SC-230M, |
| Polyquaternium-1 | Vinylpyrrolidon/dimethylaminoethyl-Methacrylat-Copolymer/Diethylsulfat-Reaktionsprodukt, |

| | |
|---|---|
| | CAS-Nr. 53633-54-8, z.B. Gafquat® 755N |
| Polyquaternium-16 | Vinylpyrrolidon/vinylimidazoliniummethochlorid-Copolymer, CAS-Nr. 29297-55-0, z.B. Luviquat® HM 552 |
| Polyquaternium-17 | CAS-Nr. 90624-75-2, z.B. Mirapol® AD-1 |
| Polyquaternium-19 | Quaternisierter wasserlöslicher Polyvinylalkohol |
| Polyquaternium-20 | in Wasser dispergierbarer quaternisierter Polyvinyloctadecylether |
| Polyquaternium-21 | Polysiloxan-polydimethyl-dimethylammoniumacetat-Copolymeres, z.B. Abil® B 9905 |
| Polyquaternium-22 | Dimethyldiallylammoniumchlorid/Acrylsäure-Copolymer, CAS-Nr. 53694-7-0, z.B. Merquat® 280 |
| Polyquaternium-24 | Polymeres quaternäres Ammoniumsalz der Hydroxyethylcellulose, Reaktionsprodukt mit einem mit Lauryldimethylammonium substituierten Epoxid, CAS-Nr. 107987-23-5, z.B. Quatrisoft® LM-200 |
| Polyquaternium-28 | Vinylpyrrolidon/Methacrylamidopropyltrimethylammoniumchlorid-Copolymer, z.B. Gafquat® HS-100 |
| Polyquaternium-29 | z.B. Lexquat® CH |
| Polyquaternium-31 | CAS-Nr. 136505-02-7, z.B. Hypan® QT 100 |
| Polyquaternium-32 | N,N,N-trimethyl-2-[(2-methyl-1-oxo-2-propenyl)oxy]-Ethanaminiumchlorid, polymer mit 2-Propenamid, CAS-Nr. 35429-19-7 |
| Polyquaternium-37 | CAS-Nr. 26161-33-1 |
| Polyquaternium-44 | Copolymeres quaternäres Ammoniumsalz bestehend aus Vinylpyrrolidone and quaternisiertem Imidazolin, z.B. Luviquat Care® |

Als bevorzugt und vorteilhaft haben sich die Polymere Polyquaternium-10, Polyquaternium-22 und Polyquaternium-44 erwiesen.

Ein Erfindungsgemäß besonders bevorzugter Filmbildner stellt Polyquarternium-10 (Ucare Polymer JR-125®, Ucare Polymer JR-400® von Amerchol) dar.

Weitere vorteilhafte Filmbildner stellen die quaternisierten Guar Gumm Derivate, wie z.B. Guar Hydroxypropyltrimonium Chlorid (Jaguar Excel®, Jaguar C 162® von Rhodia) und/oder Cellulose Derivate dar.

Die angegebenen Stoffe geben nur eine Auswahl der Möglichkeiten.
Erfindungsgemäße Zubereitungen können als Dusch- und Badepeelings verwendet werden.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Als Schaumverstärker können Tenside verwendet werden:

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielsweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| $RNH_2^+CH_2CH_2COOH\ X^-$ | (bei pH=2) | $X^-$ = beliebiges Anion, z.B. $Cl^-$ |
| $RNH_2^+CH_2CH_2COO^-$ | (bei pH=7) | |
| $RNHCH_2CH_2COO^-\ B^+$ | (bei pH=12) | $B^+$ = beliebiges Kation, z.B. $Na^+$ |

Typisch für nicht-ionische Tenside sind Polyether-Ketten. Nicht-ionische Tenside bilden in wäßrigem Medium keine Ionen.

Vorteilhaft zu verwendende anionische Tenside sind

Sulfonsäuren und Salze, wie

1. Acyl-isethionate, z.B. Natrium-/ Ammoniumcocoyl-isethionat,

2. Alkylarylsulfonate,

3. Alkylsulfonate, beispielsweise Natriumcocosmonoglyceridsulfat, Natrium $C_{12-14}$ Olefin-sulfonat, Natriumlaurylsulfoacetat und Magnesium PEG-3 Cocamidsulfat,

4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.

sowie

Schwefelsäureester, wie

1. Alkylethersulfat, beispielsweise Natrium-, Ammonium-, Magnesium-, MIPA-, TIPA-Laurethsulfat, Natriummyrethsulfat und Natrium $C_{12-13}$-Parethsulfat,

2. Alkylsulfate, beispielsweise Natrium-, Ammonium- und TEA-Laurylsulfat.

Vorteilhaft zu verwendende nicht-ionische Tenside sind

1. Alkohole,

2. Alkanolamide, wie Cocamide MEA/ DEA/ MIPA,

3. Ester, die durch Veresterung von Carbonsäuren mit Ethylenoxid, Glycerin, Sorbitan oder anderen Alkoholen entstehen,

4. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.

Vorteilhaft ist ferner die Verwendung einer Kombination von anionischen und/oder amphoteren Tensiden mit einem oder mehreren nicht-ionischen Tensiden.

Eine gegebenenfalls gewünschte Ölkomponente der kosmetischen oder dermatologischen Reinigungszubereitungen - beispielsweise in Form von Reinigungsemulsionen - im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölkomponente vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur

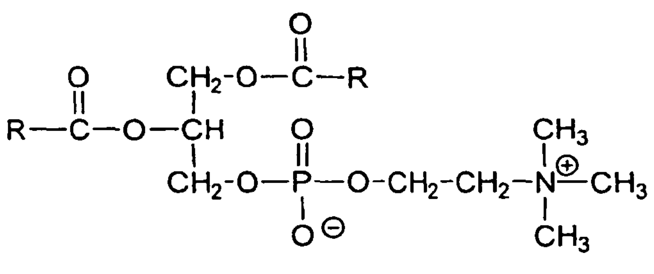

auszeichnen, wobei R' und R" typischerweise unverzweigte aliphatische Reste mit 15 oder 17 Kohlenstoffatomen und bis zu 4 cis-Doppelbindungen darstellen.

Beispiele

Die in der Rezeptur angegebenen Gehalte sind die Aktivgehalte der jeweiligen Rohstoffe.

| **Beispielrezepturen** | | | | | | |
|---|---|---|---|---|---|---|
| | **1** | **2** | **3** | **4** | **5** | **6** |
| Natriumlaurethsulfat | 11,0 | 6,0 | - | 8,0 | - | - |
| Cocamidopropylbetain | - | 4,0 | - | 2,0 | 2,0 | 4,0 |
| Natrium Myristylethersulfat | - | - | 6,0 | - | 7,0 | 7,0 |
| Alkylpolyglucoside | - | 3,0 | - | - | 2,0 | - |
| Carbopol ETD 2020 | 0,7 | - | 0,6 | 0,5 | - | 0,4 |
| Carbopol 1328 | - | 0,7 | - | - | 1,0 | - |
| PEG-7 Glyceryl Cocoat | - | - | 1,0 | 1,0 | - | - |
| Paraffinöl | 34,0 | 30,0 | 45,0 | 40,0 | 25,0 | 35,0 |
| Sojaöl | 8,0 | 12,0 | - | 5,0 | 15,0 | 10,0 |
| Mandelöl | - | 1,0 | - | 1,0 | - | 1,0 |
| Jojobaöl | - | 0,5 | 1,0 | - | 1,0 | - |
| Reisöl | 0,5 | - | 1,0 | 0,5 | - | - |
| Polyquaternium - 7 | - | - | 0,1 | - | - | - |
| Polyquaternium - 10 | - | - | - | - | 0,2 | - |
| Polyethylen | 2,9 | 3,0 | - | 5,0 | 3,0 | 5,0 |
| Kalziumcarbonat | - | 2,0 | - | - | - | 7,0 |
| Nussschalen | - | - | 1,0 | - | 3,0 | - |
| Reiskleiewachs | 0,1 | - | 0,5 | - | - | - |
| Unispheres UEL-611 | - | - | - | - | 0,2 | - |
| Methylparaben | 0,4 | 0,3 | 0,1 | 0,3 | 0,2 | 0,4 |
| Ethylparaben | 0,1 | - | 0,1 | - | 0,1 | - |
| Propylparaben | 0,2 | 0,2 | 0,1 | 0,2 | 0,2 | 0,2 |
| Butylparaben | 0,1 | - | 0,1 | - | - | - |
| Isobutylparaben | 0,1 | - | 0,1 | - | 0,1 | - |
| Phenoxyethanol | 0,6 | 0,6 | 0,5 | 0,6 | 0,5 | 0,6 |
| Butylhydroxyethanol | 0,1 | 0,1 | 0,1 | 0,1 | 0,1 | - |
| Natriumhydroxid | q.s. | q.s. | q.s. | q.s. | q.s. | q.s. |
| Parfüm | 1,2 | 0,8 | 1,5 | 1,0 | 1,2 | 1,8 |
| Wasser | ad 100 | ad 100 | ad 100 | ad 100 | ad 100 | ad 100 |
| Parameter | Rezeptur 1 | Rezeptur 2 | Rezeptur 3 | Rezeptur 4 | Rezeptur 5 | Rezeptur 6 |
| Fließgrenze [Pa] | 12,5 | 33,0 | 8,0 | 2,0 | 82,0 | 1,7 |
| tan Delta | 0,2 | 0,2 | 0,3 | 0,5 | 0,2 | 0,4 |
| Viskosität [dPas] | 55 | 80 | 40 | 42 | 122 | 79 |
| $K_{sed.}$ | 1,0 | 1,0 | 1,1 | 1,1 | 1,0 | 1,1 |

(fortgesetzt)

| Parameter | Rezeptur 1 | Rezeptur 2 | Rezeptur 3 | Rezeptur 4 | Rezeptur 5 | Rezeptur 6 |
|---|---|---|---|---|---|---|
| Partikeldichte [g/$cm^3$] Polyethylen | 0,95 | 0,95 | | 0,95 | 0,95 | 0,95 |
| Reiskleiewachs | 0,99 | | 0,99 | | | |
| Kalziumkarbonat | | 2,7 | | | | 2,7 |
| Wallnussschalen | | | 1,3 | | 1,3 | |
| Partikelgröße [μm] Polyethylen | 100-500 | 100-500 | | 100-500 | 100-500 | 100-500 |
| Reiskleiewachs | 200-850 | | 200-850 | | | |
| Kalziumkarbonat | | 100-300 | | | | 100-300 |
| Wallnussschalen | | | 300-600 | | 300-600 | |

**Patentansprüche**

1. Kosmetische Reinigungsemulsion enthaltend Öl, Wasser, Tenside, Schleifkörper und Verdicker wobei als Verdicker 0,3 bis 1,0 Gew.% von Acryl- und/oder Methacrylsäure und/oder deren Derivaten abgeleitetes Polymer und als Schleifkörper solche mit einer durchschnittlichen Partikelgröße von 100μm - 1500μm verwendet werden, **dadurch gekennzeichnet, dass** sie mehr als 10 Gew.% Paraffinum Liquidum enthält.

2. Emulsion nach Patentanspruch 1 **dadurch gekennzeichnet, dass** der Partikel-Sedimentationskoeffizient nach einer viermonatigen Lagerung bei 50°C weniger als 1,2 beträgt.

3. Emulsion nach einem der vorangehenden Patentansprüche **dadurch gekennzeichnet, dass** Schleifkörper mit einem Schmelzpunkt unterhalb 700 °C verwendet werden.

4. Emulsion nach einem der vorangehenden Patentansprüche **dadurch gekennzeichnet, dass** Schleifkörper mit einer Dichte größer 0,9 g $cm^{-3}$ verwendet werden.

5. Emulsion nach einem der vorangehenden Patentansprüche **dadurch gekennzeichnet, dass** mindestens 0,5 Gew.% und höchstens 15 Gew.% Schleifkörper verwendet werden.

6. Emulsion nach einem der vorangehenden Patentansprüche **dadurch gekennzeichnet, dass** als Verdicker ein Alkylacrylat-Polymer verwendet wird.

7. Emulsion nach einem der vorangehenden Patentansprüche **dadurch gekennzeichnet, dass** ihre Viskosität von 3600 mPas bis 15000 mPas beträgt, ermittelt mit Rotationsviskosimeter VIskotester VT02 der Firma Haake mit jeweils dem Messbereich adäquater Spindel.

8. Emulsion nach einem der vorangehenden Patentansprüche **dadurch gekennzeichnet, dass** ihre Fließgrenze 0,5 Pa bis 300 Pa beträgt.

9. Emulsion nach einem der vorangehenden Patentansprüche **dadurch gekennzeichnet, dass** sie einen tan delta zwischen 0,01 und 0,8 aufweist.

10. Emulsion nach einem der vorangehenden Patentansprüche **dadurch gekennzeichnet, dass** sie höchstens 50 Gew.% Öl enthält.

11. Emulsion nach einem der vorangehenden Patentansprüche **dadurch gekennzeichnet, dass** sie mindestens 5 Gew.% Tenside enthält.

12. Emulsion nach einem der vorangehenden Patentansprüche **dadurch gekennzeichnet, dass** sie mindestens ein anionisches Tensid enthält.

13. Emulsion nach einem der vorangehenden Patentansprüche **dadurch gekennzeichnet, dass** sie mehr als 5 Gew. % ungesättigter Lipide enthält.

14. Verwendung von Emulsionen nach einem der vorangehenden Patentansprüche zur Reinigung und Pflege von keratinischen Strukturen.

## Claims

1. Cosmetic cleaning emulsion comprising oil, water, surfactants, abrasive bodies and thickener, where the thickener used is 0.3 to 1.0% by weight of polymer derived from acrylic acid and/or methacrylic acid and/or derivatives thereof, and the abrasive bodies used are those with an average particle size of 100 μm-1500 μm, **characterized in that** it comprises more than 10% by weight of paraffinum liquidum.

2. Emulsion according to Patent Claim 1, **characterized in that** the particle sedimentation coefficient after storage for four months at 50°C is less than 1.2.

3. Emulsion according to one of the preceding patent claims, **characterized in that** abrasive bodies with a melting point below 700°C are used.

4. Emulsion according to one of the preceding patent claims, **characterized in that** abrasive bodies with a density of greater than 0.9 g $cm^{-3}$ are used.

5. Emulsion according to one of the preceding patent claims, **characterized in that** at least 0.5% by weight and at most 15% by weight of abrasive bodies are used.

6. Emulsion according to one of the preceding patent claims, **characterized in that** the thickener used is an alkyl acrylate polymer.

7. Emulsion according to one of the preceding patent claims, **characterized in that** its viscosity is from 3600 mPas to 15 000 mPas, determined using a Viskotester VT02 rotary viscometer from Haake with a spindle suitable in each case for the measurement range.

8. Emulsion according to one of the preceding patent claims, **characterized in that** its yield point is 0.5 Pa to 300 Pa.

9. Emulsion according to one of the preceding patent claims, **characterized in that** it has a tan delta between 0.01 and 0.8.

10. Emulsion according to one of the preceding patent claims, **characterized in that** it comprises at most 50% by weight of oil.

11. Emulsion according to one of the preceding patent claims, **characterized in that** it comprises at least 5% by weight of surfactants.

12. Emulsion according to one of the preceding patent claims, **characterized in that** it comprises at least one anionic surfactant.

13. Emulsion according to one of the preceding patent claims, **characterized in that** it comprises more than 5% by weight of unsaturated lipids.

14. Use of emulsions according to one of the preceding patent claims for the cleaning and care of keratin structures.

## Revendications

1. Emulsion de nettoyage cosmétique comprenant de l'huile, de l'eau, des agents tensioactifs, des corps abrasifs et des agents épaississants, où on utilise, en tant qu'agents épaississants, de 0,3 à 1,0 % en poids d'un polymère dérivé de l'acide acrylique et/ou méthacrylique et/ou de leurs dérivés, et en tant que corps abrasifs, ceux présentant

une taille moyenne de particules de 100 µm à 1500 µm, **caractérisée en ce qu'**elle comprend plus de 10 % en poids de paraffine liquide.

2. Emulsion selon la revendication 1, **caractérisée en ce que** le coefficient de sédimentation de particules après un stockage pendant quatre mois à 50°C est inférieur à 1,2.

3. Emulsion selon l'une quelconque des revendications précédentes, **caractérisée en ce que** l'on utilise des corps abrasifs présentant un point de fusion inférieur à 700°C.

4. Emulsion selon l'une quelconque des revendications précédentes, **caractérisée en ce que** l'on utilise des corps abrasifs présentant une densité supérieure à 0,9 g $cm^{-3}$.

5. Emulsion selon l'une quelconque des revendications précédentes, **caractérisée en ce que** l'on utilise au moins 0,5 % en poids et au plus 15 % en poids de corps abrasifs.

6. Emulsion selon l'une quelconque des revendications précédentes, **caractérisée en ce que** l'on utilise un polymère d'acrylate d'alkyle en tant qu'agent épaississant.

7. Emulsion selon l'une quelconque des revendications précédentes, **caractérisée en ce que** sa viscosité est de 3600 mPas à 15 000 mPas, déterminée par un viscosimètre rotatif Viskotester VT02 de Haake avec une broche adéquate dans chaque cas pour la mesure.

8. Emulsion selon l'une quelconque des revendications précédentes, **caractérisée en ce que** son seuil d'écoulement est de 0,5 Pa à 300 Pa.

9. Emulsion selon l'une quelconque des revendications précédentes, **caractérisée en ce qu'**elle présente un tan delta compris entre 0,01 et 0,8.

10. Emulsion selon l'une quelconque des revendications précédentes, **caractérisée en ce qu'**elle comprend au plus 50 % en poids d'huile.

11. Emulsion selon l'une quelconque des revendications précédentes, **caractérisée en ce qu'**elle comprend au moins 5 % en poids d'agents tensioactifs.

12. Emulsion selon l'une quelconque des revendications précédentes, **caractérisée en ce qu'**elle comprend au moins un agent tensioactif anionique.

13. Emulsion selon l'une quelconque des revendications précédentes, **caractérisée en ce qu'**elle comprend plus de 5 % en poids de lipides insaturés.

14. Utilisation d'émulsions selon l'une quelconque des revendications précédentes pour le nettoyage et le soin de structures kératiniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10318526
- EP 1166772 A
- DE 10153023
- DE 10161171
- WO 9632092 A
- FR 2731616


### In der Beschreibung aufgeführte Nicht-Patentliteratur


- *CHEMICAL ABSTRACTS,* 63451-27-4